# EUROPEAN PATENT SPECIFICATION

(11) **EP 2 686 447 B1**
(45) Date of publication and mention of the grant of the patent: **12.04.2017**
(21) Application number: 12757820.1
(22) Date of filing: 16.02.2012
(51) Int. Cl.: C12Q 1/68, C40B 30/04, C40B 40/06, G01N 33/574

(54) **PROGNOSTIC MARKER SETS FOR PROSTATE CANCER**
PROGNOSTISCHE MARKER-SETS FÜR PROSTATAKREBS
JEUX DE MARQUEURS DE PRONOSTIC DU CANCER DE LA PROSTATE

(30) Priority: 14.03.2011 US 201161452439 P
(43) Date of publication of application: 22.01.2014
(73) Proprietor: National Research Council of Canada, Ottawa, Ontario K1A 0R6 (CA)
(72) Inventor: WANG, Edwin, Laval, Quebec H7Y 2G9 (CA); LI, Jie, Montreal, Quebec H3W 3C8 (CA); O.CONNOR-MCCOURT, Maureen, Beaconsfield, Quebec H9W 1P2 (CA)
(74) Representative: Jostarndt Patentanwalts-AG
(86) International application number: PCT/CA2012/000141
(87) International publication number: WO 2012/122626

(56) References cited:
- WO-A1-2010/118520
- WO-A2-2006/091776
- US-A1- 2010 233 691
- JIE LI ET AL: "Identification of high-quality cancer prognostic markers and metastasis network modules", NATURE COMMUNICATIONS, vol. 1, no. 4, 13 July 2010 (2010-07-13), pages 1-8, XP055152017, ISSN: 2041-1723, DOI: 10.1038/ncomms1033
- TOHRU NAKAGAWA ET AL: "A Tissue Biomarker Panel Predicting Systemic Progression after PSA Recurrence Post-Definitive Prostate Cancer Therapy", PLOS ONE, vol. 3, no. 5, 28 May 2008 (2008-05-28), page e2318, XP055010915, DOI: 10.1371/journal.pone.0002318
- GLINSKY GENNADI V ET AL: "Microarray analysis identifies a death-from-cancer signature predicting therapy failure in patients with multiple types of cancer", JOURNAL OF CLINICAL INVESTIGATION, AMERICAN SOCIETY FOR CLINICAL INVESTIGATION, US, vol. 115, no. 6, 1 June 2005 (2005-06-01), pages 1503-1521, XP002460132, ISSN: 0021-9738, DOI: 10.1172/JCI23412
- LAPOINTE J ET AL: "Gene expression profiling identifies clinically relevant subtypes ofprostate cancer", PROCEEDINGS OF THE NATIONAL ACADEMY OF SCIENCES, NATIONAL ACADEMY OF SCIENCES, US, vol. 101, no. 3, 1 January 2004 (2004-01-01), pages 811-816, XP002497761, ISSN: 0027-8424, DOI: 10.1073/PNAS.0304146101
- SINGH, D. ET AL.: 'Gene expression correlates of clinical prostate cancer behavior.' CANCER CELL. vol. 1, no. 2, March 2002, pages 203 - 209, XP003010223
- GLINSKY, G. V. ET AL.: 'Gene expression profiling predicts clinical outcome of prostate cancer.' JOURNAL OF CLINICAL INVESTIGATION. vol. 113, no. 6, March 2004, pages 913 - 923, XP002395330
- NAKAGAWA, T. ET AL.: 'A tissue biomarker panel predicting systemic progression after PSA recurrence post-definitive prostate cancer therapy. art e2318' PLOS ONE. vol. 3, no. 5, 28 May 2008, XP055010915
- CHEVILLE, C. J. ET AL.: 'Gene panel model predictive of outcome in men at high-risk of systemic progression and death from prostate cancer after radical retropubic prostatectomy.' JOURNAL OF CLINICAL ONCOLOGY. vol. 26, no. 24, 20 August 2008, pages 3930 - 3936, XP055125419

## Description

### Field of the Invention

The present invention is related to prostate cancer, more particularly to methods and markers for predicting prostate cancer risk.

### Background of the Invention

There has been significant effort in the past directed to the diagnosis of prostate cancer. The well known prostate specific antigen (PSA) test is one diagnostic test. Another test (Belacel 2010) describes the use of eight different marker genes for diagnosing prostate cancer. Although a variety of tests have been developed for diagnosing prostate cancer, there have been relatively few efforts directed to developing prognostic tests for predicting low-risk patients in order to determine the proper treatment regime for patients diagnosed with prostate cancer. Two large scale studies of prostate cancer recently showed that there is significant over-diagnosis and overtreatment of prostate cancer patients (Andriole 2009; Schröder 2009). Many prostate cancer patients suffer from the side effects of treatment and society is bearing the related costs. Most of these treatments are unnecessary.

Recently, an algorithm (Multiple Survival Screening (MSS)) has been developed for identifying high-quality cancer prognostic markers and this algorithm was applied for identifying robust marker sets for breast cancer prognosis (Li 2010; Wang 2010).

There is a need to find new markers and develop new tests which are able to more accurately predict low-risk patients for prostate cancer who should receive little or no treatment.

### Summary of the Invention

It has now been found that prostate cancer marker sets consisting of particular genes differentially expressed in prostate tumours advantageously provide improved accuracy of prostate cancer prognosis. The prostate cancer marker sets of the present invention, one of which consists of 30 genes related to apoptosis, one of which consists of 22 genes related to cell cycle and one of which consists of 30 genes related to response to external stimulus, may be used in a clinical setting to provide information about the likelihood of a prostate cancer patient to survive without treatment (i.e. whether the prostate tumour is "good" or "bad").

In one aspect of the present invention, there is provided a method of assessing likelihood of a patient having a prostate tumour benefiting from prostate cancer treatment, the method comprising: obtaining a sample of the prostate tumour or an extract thereof having message RNA therein of the patient; determining a gene expression profile of the sample for genes of a gene marker set; and, comparing the gene expression profile of the sample to standardized "good" and "bad" profiles of the marker set to determine whether the gene expression profile of the sample predicts that the tumour is "good" or "bad", wherein "good" indicates that the patient is predicted to be at low-risk and would not likely benefit from prostate cancer treatment, "bad" indicates that the patient is predicted to be at high-risk and would likely benefit from prostate cancer treatment, and the gene marker set is Set 1, Set 2 and Set 3, wherein
Set 1 consists of apoptosis-related genes as follows:

| Gene | EntrezGene ID | Full Name of Gene |
|---|---|---|
| COL4A3 | 1285 | type IV collagen |
| BIRC5 | 332 | baculoviral IAP repeat containing 5 |
| TOP2A | 7153 | topoisomerase (DNA) II alpha |
| CDC2 | 983 | cyclin-dependent kinase 1 (CDK1) |
| NRAS | 4893 | Neuroblastoma RAS viral (v-ras) oncogene homolog |
| GAS1 | 2619 | growth arrest-specific 1 |
| LIG4 | 3981 | ligase IV, DNA, ATP-dependent |
| OSM | 5008 | oncostatin M |
| PML | 5371 | promyelocytic leukemia |
| TP53 | 7157 | tumour protein p53 |
| NF1 | 4763 | neurofibromin 1 |
| SIAH1 | 6477 | seven in absentia homolog 1 (Drosophila) |
| MALT1 | 10892 | mucosa associated lymphoid tissue lymphoma translocation gene 1 |
| KIT | 3815 | v-kit Hardy-Zuckerman 4 feline sarcoma viral oncogene homolog |
| RHOA | 387 | ras homolog gene family, member A |
| ESR1 | 2099 | estrogen receptor 1 |
| | | |
| RARB | 5915 | retinoic acid receptor, beta |
| VAV1 | 7409 | vav 1 guanine nucleotide exchange factor |
| WRN | 7486 | Werner syndrome, RecQ helicase-like |
| TNFRSF10A | 8797 | tumour necrosis factor receptor superfamily, member 10a |
| RIPK1 | 8737 | receptor (TNFRSF)-interacting serine-threonine kinase 1 |
| ABL1 | 25 | c-abl oncogene 1, non-receptor tyrosine kinase |
| TERT | 7015 | telomerase reverse transcriptase |
| GLI3 | 2737 | GLI family zinc finger 3 |
| JUN | 3725 | jun proto-oncogene |
| NFKBIA | 4792 | nuclear factor of kappa light polypeptide gene enhancer in B-cells inhibitor, alpha |
| LCK | 3932 | lymphocyte-specific protein tyrosine kinase |
| CASP3 | 836 | caspase 3, apoptosis-related cysteine peptidase |
| E2F2 | 1870 | E2F transcription factor 2 |
| LTA | 4049 | lymphotoxin alpha (TNF superfamily, member 1) |

Set 2 consists of cell cycle-related genes as follows:

| Gene Name | EntrezGene ID | Description |
|---|---|---|
| BCL2 | 596 | B-cell CLL/lymphoma 2 |
| RAD51 | 5888 | RAD51 homolog (RecA homolog, E. coli) (S. cerevisiae) |
| CDKN2B | 1030 | cyclin-dependent kinase inhibitor 2B (p15, inhibits CDK4) |
| GML | 2765 | glycosylphosphatidylinositol anchored molecule like protein |
| E2F1 | 1869 | E2F transcription factor 1 |
| IKZF1 | 10320 | IKAROS family zinc finger 1 (Ikaros) |
| BLM | 641 | Bloom syndrome, RecQ helicase-like |
| ABL1 | 25 | c-abl oncogene 1, non-receptor tyrosine kinase |
| LIG4 | 3981 | ligase IV, DNA, ATP-dependent |
| CCNA2 | 890 | cyclin A2 |
| NUMA1 | 4926 | nuclear mitotic apparatus protein 1 |
| CCNC | 892 | cyclin C |
| RBL2 | 5934 | retinoblastoma-like 2 (p130) |
| LTA | 4049 | lymphotoxin alpha (TNF superfamily, member 1) |
| ERCC2 | 2068 | excision repair cross-complementing rodent repair deficiency, complementation group 2 |
| CASP3 | 836 | caspase 3, apoptosis-related cysteine peptidase |
| TP53 | 7157 | tumour protein p53 |
| | | |
| RAD54L | 8438 | RAD54-like (S. cerevisiae) |
| CCND3 | 896 | cyclin 03 |
| WEE1 | 7465 | WEE1 homolog (S. pombe) |
| BIRC5 | 332 | baculoviral IAP repeat containing 5 |
| HDAC1 | 3065 | histone deacetylase 1 |

Set 3 consists of response to external stimulus-related genes as follows:

| Gene Name | EntrezGene ID | Description |
|---|---|---|
| COL4A3 | 1285 | Type IV collagen |
| TOP2A | 7153 | topoisomerase (DNA) II alpha |
| CDC2 | 983 | cyclin-dependent kinase 1 (CDK1) |
| LYN | 4067 | v-yes-1 Yamaguchi sarcoma viral related oncogene homolog |
| PXN | 5829 | paxillin |
| NTRK3 | 4916 | neurotrophic tyrosine kinase, receptor, type 3 |
| PDGFRA | 5156 | platelet-derived growth factor receptor, alpha polypeptide |
| NRAS | 4893 | Neuroblastoma RAS viral (v-ras) oncogene homolog |
| CHEK1 | 1111 | CHK1 checkpoint homolog (S. pombe) |
| PARP1 | 142 | poly (ADP-ribose) polymerase 1 |
| KIT | 3815 | v-kit Hardy-Zuckerman 4 feline sarcoma viral oncogene homolog |
| TGFBR3 | 7049 | transforming growth factor, beta receptor 111 |
| CCNA2 | 890 | cyclin A2 |
| NF1 | 4763 | neurofibromin 1 |
| MAPK10 | 5602 | mitogen-activated protein kinase 10 |
| CD9 | 928 | CD9 molecule |
| ESR1 | 2099 | estrogen receptor 1 |
| FRAP1 | 2475 | mechanistic target of rapamycin (serine/threonine kinase) (MTOR) |
| PML | 5371 | promyelocytic leukemia |
| ABL1 | 25 | c-abl oncogene 1, non-receptor tyrosine kinase |
| TP53 | 7157 | tumour protein p53 |
| LIG4 | 3981 | ligase IV, DNA, ATP-dependent |
| WEE1 | 7465 | WEE1 homolog (S. pombe) |
| SYK | 6850 | spleen tyrosine kinase |
| MALT1 | 10892 | mucosa associated lymphoid tissue lymphoma translocation gene 1 |
| PTCH1 | 5727 | patched 1 |
| CASP3 | 836 | caspase 3, apoptosis-related cysteine peptidase |
| BLM | 641 | Bloom syndrome, RecQ helicase-like |
| FYN | 2534 | FYN oncogene related to SRC, FGR, YES |
| WRN | 7486 | Werner syndrome, RecQ helicase-like |

The genes in the prostate cancer marker sets of the present invention are individually known and are individually known to be differentially expressed in prostate tumour cells. How they are differentially expressed and whether their differential expression generally correlates to "good" or "bad" tumours can also be determined from publicly available datasets. However, the specific combination of the genes in each marker set of the present invention unexpectedly provides for more robust marker sets having improved prognostic accuracy for prostate cancer survival. The marker sets of the present invention consisting of the specific combination of genes that gives rise to the improved prognostic accuracy may be generated using the Multiple Survival Screening (MSS) method previously developed (Li 2010; Wang 2010).

The sample comprises a sample of the prostate tumour of the patient or an extract thereof, which contains the genes in the marker set or message RNA that hybridizes to the genes in the marker set. Preferably, the sample comprises a sample of the prostate tumour of the patient.

According to the invention, all three sets are used together to make risk predictions. Thus, gene expression profiles of the sample are determined for the genes in each of Sets 1, 2 and 3. In this case, the gene expression profiles are compared to standardized "good" and "bad" profiles of each respective gene marker set to determine whether each of the gene expression profiles predicts that the tumour is "good" or "bad". If all three marker sets predict that the tumour is "good" then the patient is predicted to be at low-risk and would not likely benefit from prostate cancer treatment. If all three marker sets predict that the tumour is "bad" then the patient is predicted to be at high-risk and would likely benefit from prostate cancer treatment. If one or two of the marker sets predict that the tumour is "good" or one or two of the marker sets predict that the tumour is "bad" then the patient is predicted to be at intermediate-risk and may or may not benefit from prostate cancer treatment. Using all three marker sets improves accuracy of the prognosis.

In a particular embodiment, each gene in the gene expression profile has a gene expression value and a modified gene expression profile is obtained by multiplying the gene expression value by its marker-factor. Standardized "good" and "bad" profiles are determined by computing standardized centroids for both "good" and "bad" classes using prediction analysis for microarrays method (Tibshirani 2002). Modified class centroids of the marker set are obtained by multiplying the standardized centroids for each class by the marker-factor. The modified gene expression profile of the sample is compared to each modified class centroid to determine the tumour is "good" or "bad". The class whose centroid is closest to the modified gene expression profile, in Pearson correlation distance, is predicted to be the class for the sample.

Gene expression profiles of a patient's prostate tumour may be readily obtained by any number of methods known in the art, for example microarray analysis, individual gene screening, etc. In a preferred embodiment, the sample is screened that against a microarray on which gene probes of the marker sets are printed. An output of the gene expression profile of the sample is preferably obtained before comparing the gene expression profile to the standardized "good" and "bad" profiles of the marker set. To obtain the output, message RNA in the sample may be hybridized to the genes on the microarray, the hybridized microarray may be scanned to get all the readouts of marker genes for the sample, the readouts may be normalized and the gene expression profile of the marker set for the sample is thereby obtained. Detailed information for making microarray gene chip, scanning and normalization of array data is generally known in the art and can be found in the publicly available literature (http://en.wikipedia.org/wiki/DNA_microarray). It is also possible to obtain the gene expression profile by RNA-sequencing and related sequencing technologies as these technologies become more accessible (http://en.wikipedia.org/wiki/RNA-Seq).

In another embodiment, the invention provides a set of gene probes consisting of the genes of the gene marker sets 1, 2 and 3 of the present invention.
Furthermore disclosed are kits or commercial packages comprising gene probes consisting of the genes of the gene marker sets 1, 2 and 3 along with instructions for obtaining a gene expression profile of a sample for the gene marker set. The kit or commercial package may further comprise instructions for comparing the gene expression profile of the sample to standardized "good" and "bad" profiles of the marker set to determine whether the gene expression profile of the sample predicts that the tumour is "good" or "bad". Preferably, the kit or commercial package comprises gene probes for all three gene marker sets of the present invention. The kit or commercial package may further comprise means for obtaining a sample of a prostate tumour having message RNA therein from a patient, for example suitable syringes, fluid and/or tissue separation means, etc. In addition to the gene probes, the kit or commercial package may further comprise reagents and/or equipment useful for screening the sample against the gene probes for obtaining the gene expression profile of the sample.
Various standard elements of such kits or commercial packages are generally known in the art.

Further features of the invention will be described or will become apparent in the course of the following detailed description.

### Brief Description of the Drawings

In order that the invention may be more clearly understood, embodiments thereof will now be described in detail by way of example, with reference to the accompanying drawings, in which:

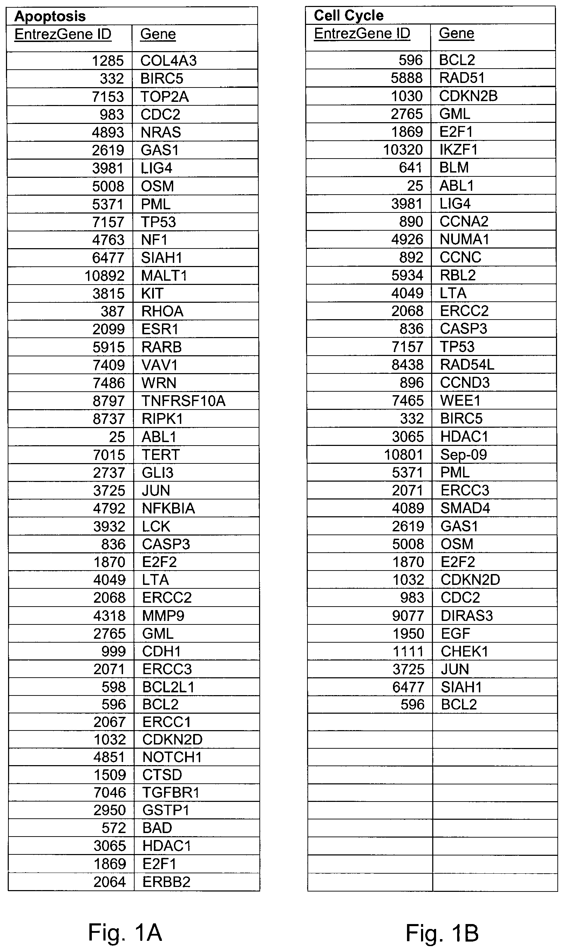

Fig. 1A provides gene names and EntrezGene ID numbers for genes in the GSE10645 prostate cancer gene expression dataset which are deposited in a public database (http://www.ncbi.nlm.nih.gov/geo/) that belong to apoptosis GO term;
Fig. 1B provides gene names and EntrezGene ID numbers for genes in the GSE10645 prostate cancer gene expression dataset that belong to cell cycle GO term; and,

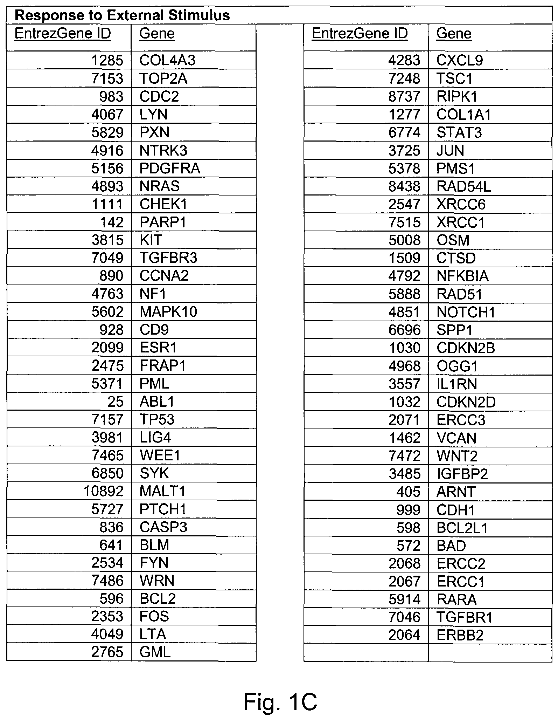

Fig. 1C provides gene names and EntrezGene ID numbers for genes in the GSE10645 prostate cancer gene expression dataset that belong to response to external stimulus GO term.

### Description of Preferred Embodiments

### Example 1: Generation of Prostate Cancer Marker Sets

To develop the prostate cancer marker sets of the present invention, the Multiple Survival Screening (MSS) method (Li 2010; Wang 2010) was used. In applying this method, a training set of 189 samples was selected from the GSE10645 GEO dataset (Nakagawa 2008). This prostate cancer gene expression dataset is from the population-based Swedish-Watchful Waiting cohort. The cohort consists of men with localized prostate cancer (clinical stage T1-T2, Mx, NO). The GSE10645 GEO dataset contains information about genes that are differentially expressed in prostate tumours. The dataset identifies whether each of these genes is up-regulated or down-regulated in tumours and correlates these genes to patient survival (i.e. "good" vs. "bad" tumours).

The 189 samples from GSE10645 were randomly divided into three groups of 63 samples, each group retaining the same proportion of "good" vs. "bad" tumours that was identified in the original GSE10645 dataset. Array-wide screening of the genes was performed on each of the three groups as described in the art (Li 2010; Wang 2010) to obtain survival genes, which are genes whose differential expression values are correlated with prostate cancer patient survivals. It is not relevant whether the expression of each gene is upregulated or downregulated so long as the differential expression is correlated to patient survival. Merging the results from each of the three groups yielded a survival gene set, which includes 133 survival genes.

Using the survival gene set, Gene Ontology (GO) analysis (using GO annotation software, David, http://david.abcc.ncifcrf.gov/) was performed to identify only those genes that belong to GO terms that are known to be associated with prostate cancer, such as apoptosis (cell death), cell adhesion, cell cycle, phosphorylation, response to external stimulus, cell motility and cell assembly. Table 1 lists the cancer-related GO term gene sets. One million distinct random-gene-sets were generated by randomly picking 30 genes from each cancer-related GO term gene set.

**Table 1**

| GO Term | Number of genes |
|---|---|
| Apoptosis | 47 |
| Cell adhesion | 68 |
| Cell cycle | 36 |
| Phosphorylation | 72 |
| Response to external stimulus | 67 |
| Cell motility | 49 |
| Cell assembly | 67 |

Of the 189 samples selected from the GSE10645 GEO dataset to form the training set, 36 random datasets were generated by randomly picking 60 samples from the training set while retaining in each random dataset the same proportion of "good" vs. "bad" tumours that was identified in the original GSE10645 dataset.

For a given GO term gene set, survival screening was then conducted using the 1 million random-gene-sets against all the 36 random datasets. For each random dataset, the statistical significance of the correlation between the expression values of each random-gene-set (30 genes) and patient survival status ("good" or "bad") was examined by Kaplan-Meier analysis by implementing the Cox-Mantel log-rank test (Cui 2007). If the P value was less than a cut-off for a survival screening using one random-gene-set against one random dataset, that random-gene-set was said to have passed. When a few thousands of random-gene-sets had passed 32 or more random datasets (the detailed parameters are shown in Table 5), the random-gene-sets that had passed were retained for further analysis. The genes in the retained random-gene-sets were then ranked based on their frequency of appearance in the passed random-gene-sets. The top 30 genes were chosen as a potential-marker-set. A similar survival screening of random-gene-sets against random datasets was performed for each of the other selected GO term gene sets.

For each GO term gene set another 1 million distinct random-gene-sets were generated and the survival screening process using the random datasets mentioned above was repeated. If the gene members for the top 30 were substantially the same as those in the potential-marker-set generated by the first screening, then the potential-marker-set is stable and can be used as a real prostate cancer marker set. If the genes for the two potential marker sets were not substantially the same, then these GO term genes are unsuitable for finding a real marker set and the potential marker set was dropped from further analysis. In some cases somewhat fewer than 30 genes may be the same in the two potential marker sets, in which case the smaller set may be designated as a marker set.

In this way, three prostate cancer marker sets were generated having stable signatures, one related to apoptosis, one related to cell cycle and one related to response to external stimulus. The genes, EntrezGene ID and full names of the genes in each of the three marker sets are given in the Tables 2-4 below. More details of each gene, including the nucleotide sequence of each gene, are known in the art and may be conveniently found in the National Center for Biotechnology Information (NCBI) Databases at http://www.ncbi.nlm.nih.gov/.

**Table 2- Marker Set Related to Apoptosis (30 genes)**

| Gene | EntrezGene ID | Full Name of Gene |
|---|---|---|
| COL4A3 | 1285 | type IV collagen |
| BIRC5 | 332 | baculoviral IAP repeat containing 5 |
| TOP2A | 7153 | topoisomerase (DNA) II alpha |
| CDC2 | 983 | cyclin-dependent kinase 1 (CDK1) |
| NRAS | 4893 | Neuroblastoma RAS viral (v-ras) oncogene homolog |
| GAS1 | 2619 | growth arrest-specific 1 |
| | | |
| LIG4 | 3981 | ligase IV, DNA, ATP-dependent |
| OSM | 5008 | oncostatin M |
| PML | 5371 | promyelocytic leukemia |
| TP53 | 7157 | tumour protein p53 |
| NF1 | 4763 | neurofibromin 1 |
| SIAH1 | 6477 | seven in absentia homolog 1 (Drosophila) |
| MALT1 | 10892 | mucosa associated lymphoid tissue lymphoma translocation gene 1 |
| KIT | 3815 | v-kit Hardy-Zuckerman 4 feline sarcoma viral oncogene homolog |
| RHOA | 387 | ras homolog gene family, member A |
| ESR1 | 2099 | estrogen receptor 1 |
| RARB | 5915 | retinoic acid receptor, beta |
| VAV1 | 7409 | vav 1 guanine nucleotide exchange factor |
| WRN | 7486 | Werner syndrome, RecQ helicase-like |
| TNFRSF10A | 8797 | tumour necrosis factor receptor superfamily, member 10a |
| RIPK1 | 8737 | receptor (TNFRSF)-interacting serine-threonine kinase 1 |
| ABL1 | 25 | c-abl oncogene 1, non-receptor tyrosine kinase |
| TERT | 7015 | telomerase reverse transcriptase |
| GLI3 | 2737 | GLI family zinc finger 3 |
| JUN | 3725 | jun proto-oncogene |
| NFKBIA | 4792 | nuclear factor of kappa light polypeptide gene enhancer in B-cells inhibitor, alpha |
| LCK | 3932 | lymphocyte-specific protein tyrosine kinase |
| CASP3 | 836 | caspase 3, apoptosis-related cysteine peptidase |
| E2F2 | 1870 | E2F transcription factor 2 |
| LTA | 4049 | lymphotoxin alpha (TNF superfamily, member 1) |

**Table 3- Marker Set Related to Cell Cycle (22 genes)**

| Gene Name | EntrezGene ID | Description |
|---|---|---|
| BCL2 | 596 | B-cell CLL/lymphoma 2 |
| RAD51 | 5888 | RAD51 homolog (RecA homolog, E. coli) (S. cerevisiae) |
| CDKN2B | 1030 | cyclin-dependent kinase inhibitor 28 (p15, inhibits CDK4) |
| GML | 2765 | glycosylphosphatidylinositol anchored molecule like protein |
| E2F1 | 1869 | E2F transcription factor 1 |
| IKZF1 | 10320 | IKAROS family zinc finger 1 (Ikaros) |
| BLM | 641 | Bloom syndrome, RecQ helicase-like |
| ABL1 | 25 | c-abl oncogene 1, non-receptor tyrosine kinase |
| LIG4 | 3981 | ligase IV, DNA, ATP-dependent |
| CCNA2 | 890 | cyclin A2 |
| NUMA1 | 4926 | nuclear mitotic apparatus protein 1 cyclin C |
| CCNC | 892 | |
| RBL2 | 5934 | retinoblastoma-like 2 (p130) |
| LTA | 4049 | lymphotoxin alpha (TNF superfamily, member 1) |
| ERCC2 | 2068 | excision repair cross-complementing rodent repair deficiency, complementation group 2 |
| CASP3 | 836 | caspase 3, apoptosis-related cysteine peptidase |
| TP53 | 7157 | tumour protein p53 |
| RAD54L | 8438 | RAD54-like (S. cerevisiae) |
| CCND3 | 896 | cyclin D3 |
| WEE1 | 7465 | WEE1 homolog (S. pombe) |
| BIRC5 | 332 | baculoviral IAP repeat containing 5 |
| HDAC1 | 3065 | histone deacetylase 1 |

**Table 4 - Marker Set Related to Response to External Stimulus (30 genes)**

| Gene Name | EntrezGene ID | Description |
|---|---|---|
| COL4A3 | 1285 | Type IV collagen |
| TOP2A | 7153 | topoisomerase (DNA) II alpha |
| CDC2 | 983 | cyclin-dependent kinase 1 (CDK1) |
| LYN | 4067 | v-yes-1 Yamaguchi sarcoma viral related oncogene homolog |
| PXN | 5829 | paxillin |
| NTRK3 | 4916 | neurotrophic tyrosine kinase, receptor, type 3 |
| PDGFRA | 5156 | platelet-derived growth factor receptor, alpha polypeptide |
| NRAS | 4893 | Neuroblastoma RAS viral (v-ras) oncogene homolog |
| CHEK1 | 1111 | CHK1 checkpoint homolog (S. pombe) |
| PARP1 | 142 | poly (ADP-ribose) polymerase 1 |
| KIT | 3815 | v-kit Hardy-Zuckerman 4 feline sarcoma viral oncogene homolog |
| TGFBR3 | 7049 | transforming growth factor, beta receptor 111 |
| CCNA2 | 890 | cyclin A2 |
| NF1 | 4763 | neurofibromin 1 |
| MAPK10 | 5602 | mitogen-activated protein kinase 10 |
| | | |
| CD9 | 928 | CD9 molecule |
| ESR1 | 2099 | estrogen receptor 1 |
| FRAP1 | 2475 | mechanistic target of rapamycin (serine/threonine kinase) (MTOR) |
| PML | 5371 | promyelocytic leukemia |
| ABL1 | 25 | c-abl oncogene 1, non-receptor tyrosine kinase |
| TP53 | 7157 | tumour protein p53 |
| LIG4 | 3981 | ligase IV, DNA, ATP-dependent |
| WEE1 | 7465 | WEE1 homolog (S. pombe) |
| SYK | 6850 | spleen tyrosine kinase |
| MALT1 | 10892 | mucosa associated lymphoid tissue lymphoma translocation gene 1 |
| PTCH1 | 5727 | patched 1 |
| CASP3 | 836 | caspase 3, apoptosis-related cysteine peptidase |
| BLM | 641 | Bloom syndrome, RecQ helicase-like |
| FYN | 2534 | FYN oncogene related to SRC, FGR, YES |
| WRN | 7486 | Werner syndrome, RecQ helicase-like |

**Table 5- Parameters for Screening of the Marker Sets**

| | Number of Passed Sample Sets | Number of Passed Gene Sets | Cut-off P value |
|---|---|---|---|
| Apoptosis | 32 | 4674 | 0.00001 |
| Cell cycle | 32 | 5548 | 0.0001 |
| Response to external stimulus | 35 | 4142 | 0.00001 |

### Example 2: Validating Effectiveness of the Marker Sets in Prostale Cancer Prognosis

The effectiveness of the three marker sets generated in Example 1 was validated against three separate GEO datasets containing prostate cancer gene expression data from sample populations. One of the three datasets against which the markers were validated was the GSE16560 dataset described above except that 261 samples from that dataset were used. The other two test datasets were GEO datasets GSE21034 (Taylor 2010) and GSE10645 (Nakagawa 2008, the validation samples marked by the authors). In all three cases, test datasets were constructed by selecting samples from the GEO datasets so that the test datasets contained 90% "good" tumours and 10% "bad" tumours, based on ultimate patient survival outcomes, in order to simulate the suggestion that over 90% of prostate cancer patients do not actually need to be treated.

To perform the validation for a given test dataset containing 'n' samples, the gene expression profile of the marker set was extracted. For each gene expression value its marker-factor was multiplied to obtain a modified gene expression profile of the testing sample. Standardized centroids were computed for both "good" and "bad" classes from n-1 samples for the marker set using the Prediction Analysis for Microarrays (PAM) method (Tibshirani 2002). The marker-factor of each gene was multiplied to the class centroids to get modified class centroids of the marker set. For predicting the recurrence of the targeted testing sample using the marker set the modified gene expression profile of the sample was compared to each of these modified class centroids. The class whose centroid that it is closest to, in Pearson correlation distance, is the predicted class for that sample. If the sample is predicted to be a "good" tumour, it is denoted as 0, otherwise it is denoted as 1. If all three marker sets predict that a particular prostate cancer sample is "good" (i.e. denoted as 0 for all 3 marker sets), the sample is assigned to low-risk group. If all three marker sets predict that a particular prostate cancer sample is "bad" (i.e. denoted as 1 for all 3 marker sets), the sample is assigned to high-risk group. If a sample is not assigned to low-risk or high-risk group, it is assigned to intermediate-risk group.

This validation process was carried out in all three of the test datasets. Table 6 shows the results for the low-risk group in comparison to the GSE16560 training set originally used to generate the three marker sets (see Example 1). As would be expected, the accuracy of the marker sets against the training set is 100%. The accuracy of the marker sets against the test datasets derived from the three GEO datasets is remarkably high.

**Table 6- Predicting Accuracy of the Marker Sets**

| Dataset | No. of Samples | Accuracy (low-risk group} |
|---|---|---|
| GSE10645 (training set) | 189 | 100% |
| GSE 16560 | 261 | 95.58% |
| GSE21034 | 140 | 99.31% |
| GSE10645 (the validation samples marked by the authors, Nakagawa 2008) | 205 | 98.24% |

The accuracy of the present marker sets can be compared to the prior art. Table 7 provides the performance of several markers and marker sets of the prior art. Table 7 is derived from Table 5 of Nakagawa 2008. The clinical models used and the nature of the various markers and marker sets listed in Table 7 below are explained in Nakagawa 2008. It is clear comparing Table 6 to Table 7 that the prognostic accuracy of the present marker sets for determining the expected survival of a prostate cancer patient is substantially greater than the prior art markers and marker sets.

**Table 7-AUC's of Prior Art Markers and Marker Sets**

| Marker or Marker Set | Probes alone | Clinical model | | |
|---|---|---|---|---|
| | | A | B | C |
| Clinical model alone | NA | 0.736 | 0.757 | 0.783 |
| Nakagawa 2008 - Final 17 gene/probe | 0.852 | 0.857 | 0.873 | 0.883 |
| Glinsky 2004 - Signature 1 | 0.665 | 0.762 | 0.776 | 0.798 |
| Glinsky 2004- Signature 2 | 0.638 | 0.764 | 0.781 | 0.798 |
| Glinsky 2004- Signature 3 | 0.669 | 0.770 | 0.788 | 0.810 |
| Glinksy 2005 | 0.729 | 0.780 | 0.800 | 0.811 |
| Lapointe 2004- Tumor Recurrence Sig. | 0.789 | 0.825 | 0.838 | 0.855 |
| Lapointe 2004- MUC1 and AZGP1 | 0.660 | 0.767 | 0.777 | 0.793 |
| Singh 2002 | 0.783 | 0.824 | 0.838 | 0.851 |
| Yu 2004 | 0.725 | 0.797 | 0.815 | 0.830 |

Andriole GL, Crawford ED, Grubb III RL, et al. (2009) Mortality Results from a Randomized Prostate-Cancer Screening Trial. The New England Journal of Medicine. 360(13), 1310-1319.
Belacel N, Cuperlovic-Culf M, Ouellette R. (2010) Molecular Method for Diagnosis of Prostate Cancer. United States Patent 7,759,060 issued July 20, 2010.
Cui Q, Ma Y, Jaramillo M, Sari H, Awan A, Yang S, Zhang S, Liu L, Lu M, O'Connor- McCourt M, Purisima EO, Wang E. (2007) A map of human cancer signaling. Molecular Systems Biology. 3:152, 13 pages.
Glinsky GV, Glinskii AB, Stephenson AJ, Hoffman RM, Gerald WL. (2004) Gene expression profiling predicts clinical outcome of prostate cancer. J Clin Invest. 113,
Glinsky GV, Berezovska 0, Glinskii AB. (2005) Microarray analysis identifies a death-from-cancer signature predicting therapy failure in patients with multiple types of cancer. J Clin Invest. 115, 1503-21.
GO annotation software, David. http://david.abcc.ncifcrf.gov/.
Lapointe J, Li C, Higgins JP, van de Rijn M, Bair E, et al. (2004) Gene expression profiling identifies clinically relevant subtypes of prostate cancer. Proc Natl Acad Sci USA. 101, 811-6.
Li J, Lenferink AEG, Deng Y, Collins C, Cui Q, Purisima EO, O'Connor-McCourt MD, Wang E. (2010) Identification of high-quality cancer prognostic markers and metastasis network modules. Nature Communications. 1:34, DOI: 10.1038/ncomms1033.
Nakagawa T, Kollmeyer TM, Merlan BW, et al. (2008) A Tissue Biomarker Panel Predicting Systemic Progression after PSA Recurrence Post-Definitive Prostate Cancer. Therapy. PLoS one. 3(5), e2318.
National Center for Biotechnology Information (NCBI) Databases. http://www.ncbi.nlm.nih.gov/.
Sboner A, Demichelis F, Calza S, et al. (2010) Molecular Sampling of Prostate Cancer: A Dilemma for Predicting Disease Progression. BMC Medical Genomics. 3-8. (GEO Gene Expression Omnibus GSE16560).
Schröder FH, Hugosson J, Roobol MJ, et al. (2009) Screening and Prostate-Cancer Mortality in a Randomized European Study. The New England Journal of Medicine. 360(13), 1320-1328.
Singh D, Febbo PG, Ross K, Jackson DG, Manola J, et al. (2002) Gene expression correlates of clinical prostate cancer behavior. Cancer Cell. 1, 203-9.
Taylor BS, Schultz N, Hieronymus H, et al. (2010) Integrative Genomic Profiling of Human Prostate Cancer. Cancer Cell. 8(1), 11-22.
Tibshirani R, Hastie T, Narasimhan B, Chu G. (2002) Diagnosis of multiple cancer types by shrunken centroids of gene expression. PNAS. 99, 6567-6572.
Wang E, Li J, Deng Y, Lenferink AEG, O'Connor-McCourt MD, Purisima EO. (2010) Process for Tumour Characteristic and Marker Set Identification, Tumour Classification and Marker Sets for Cancer. International Patent Application WO 2010/118520 published October 21, 2010.
Wikipedia, the free encyclopedia. (2010a) DNA Microarray. http://en.wikipedia.org./wiki/DNA_microarray.
Wikipedia, the free encyclopedia. (2010b) RNA-Seq. http://en.wikipedia.org/wiki/RNA-Seq.
Yu YP, Landsittel D, Jing L, Nelson J, Ren B, et al. (2004) Gene expression alterations in prostate cancer predicting tumour aggression and preceding development of malignancy. J Clin Oncol. 22, 2790-9.

## Claims

1. A method of assessing likelihood of a patient having a prostate tumour benefiting from prostate cancer treatment, the method comprising: obtaining a sample of the prostate tumour or an extract thereof having message RNA therein of the patient; determining a gene expression profile of the sample for genes of each of three gene marker sets; and, comparing the gene expression profiles of the sample to standardized "good" and "bad" profiles of each respective gene marker set to determine whether each of the gene expression profiles predicts that the tumour is "good" or "bad",
wherein
if all three marker sets predict that the tumour is "good" then the patient is predicted to be at low-risk and would not likely benefit from prostate cancer treatment; if all three marker sets predict that the tumour is "bad" then the patient is predicted to be at high-risk and would likely benefit from prostate cancer treatment; and if one or two of the marker sets predict that the tumour is "good" or one or two of the marker sets predict that the tumour is "bad" then the patient is predicted to be at intermediate-risk and may or may not benefit from prostate cancer treatment,
and wherein the gene marker sets are Set 1, Set 2 and Set 3, where
Set 1 consists of apoptosis-related genes as follows:
| Gene | EntrezGene ID | Full Name of Gene |
|---|---|---|
| COL4A3 | 1285 | type IV collagen |
| BIRC5 | 332 | baculoviral IAP repeat containing 5 |
| TOP2A | 7153 | topoisomerase (DNA) II alpha |
| CDC2 | 983 | cyclin-dependent kinase 1 (CDK1) |
| NRAS | 4893 | neuroblastoma RAS viral (v-ras) oncogene homolog |
| GAS1 | 2619 | growth arrest-specific 1 |
| LIG4 | 3981 | ligase IV, DNA, ATP-dependent |
| OSM | 5008 | oncostatin M |
| PML | 5371 | promyelocytic leukemia |
| TP53 | 7157 | tumour protein p53 |
| NF1 | 4763 | neurofibromin 1 |
| SIAH1 | 6477 | seven in absentia homolog 1 (Drosophila) |
| MALT1 | 10892 | mucosa associated lymphoid tissue lymphoma translocation gene 1 |
| KIT | 3815 | v-kit Hardy-Zuckerman 4 feline sarcoma viral oncogene homolog |
| RHOA | 387 | ras homolog gene family, member A |
| ESR1 | 2099 | estrogen receptor 1 |
| RARB | 5915 | retinoic acid receptor, beta |
| VAV1 | 7409 | vav 1 guanine nucleotide exchange factor |
| WRN | 7486 | Werner syndrome, RecQ helicase-like |
| TNFRSF10A | 8797 | tumour necrosis factor receptor superfamily, member 10a |
| RIPK1 | 8737 | receptor (TNFRSF)-interacting serine-threonine kinase 1 |
| ABL1 | 25 | c-abl oncogene 1, non-receptor tyrosine kinase |
| TERT | 7015 | telomerase reverse transcriptase |
| GLI3 | 2737 | GLI family zinc finger 3 |
| JUN | 3725 | jun proto-oncogene |
| NFKBIA | 4792 | nuclear factor of kappa light polypeptide gene enhancer in B-cells inhibitor, alpha |
| LCK | 3932 | lymphocyte-specific protein tyrosine kinase |
| CASP3 | 836 | caspase 3, apoptosis-related cysteine peptidase |
| E2F2 | 1870 | E2F transcription factor 2 |
| LTA | 4049 | lymphotoxin alpha (TNF superfamily, member 1) |
Set 2 consists of cell cycle-related genes as follows:
| Gene Name | EntrezGene ID | Description |
|---|---|---|
| BCL2 | 596 | B-cell CLL/lymphoma 2 |
| RAD51 | 5888 | RAD51 homolog (RecA homolog, E. coli) (S. cerevisiae) |
| CDKN2B | 1030 | cyclin-dependent kinase inhibitor 2B (p15, inhibits CDK4) |
| GML | 2765 | glycosylphosphatidylinositol anchored molecule like protein |
| E2F1 | 1869 | E2F transcription factor 1 |
| IKZF1 | 10320 | IKAROS family zinc finger 1 (Ikaros) |
| BLM | 641 | Bloom syndrome, RecQ helicase-like |
| ABL1 | 25 | c-abl oncogene 1, non-receptor tyrosine kinase |
| LIG4 | 3981 | ligase IV, DNA, ATP-dependent |
| CCNA2 | 890 | cyclin A2 |
| NUMA1 | 4926 | nuclear mitotic apparatus protein 1 |
| CCNC | 892 | cyclin C |
| RBL2 | 5934 | retinoblastoma-like 2 (p130) |
| LTA | 4049 | lymphotoxin alpha (TNF superfamily, member 1) |
| ERCC2 | 2068 | excision repair cross-complementing rodent repair deficiency, complementation group 2 |
| CASP3 | 836 | caspase 3, apoptosis-related cysteine peptidase |
| TP53 | 7157 | tumour protein p53 |
| RAD54L | 8438 | RAD54-like (S. cerevisiae) |
| CCND3 | 896 | cyclin D3 |
| WEE1 | 7465 | WEE1 homolog (S. pombe) |
| BIRC5 | 332 | baculoviral IAP repeat containing 5 |
| HDAC1 | 3065 | histone deacetylase 1 |
Set 3 consists of response to external stimulus-related genes as follows:
| Gene Name | EntrezGene ID | Description |
|---|---|---|
| COL4A3 | 1285 | Type IV collagen |
| TOP2A | 7153 | topoisomerase (DNA) II alpha |
| CDC2 | 983 | cyclin-dependent kinase 1 (CDK1) |
| LYN | 4067 | v-yes-1 Yamaguchi sarcoma viral related oncogene homolog |
| PXN | 5829 | paxillin |
| NTRK3 | 4916 | neurotrophic tyrosine kinase, receptor, type 3 |
| PDGFRA | 5156 | platelet-derived growth factor receptor, alpha polypeptide |
| NRAS | 4893 | neuroblastoma RAS viral (v-ras) oncogene homolog |
| CHEK1 | 1111 | CHK1 checkpoint homolog (S. pombe) |
| PARP1 | 142 | poly (ADP-ribose) polymerase 1 |
| KIT | 3815 | v-kit Hardy-Zuckerman 4 feline sarcoma viral oncogene homolog |
| TGFBR3 | 7049 | transforming growth factor, beta receptor III |
| CCNA2 | 890 | cyclin A2 |
| NF1 | 4763 | neurofibromin 1 |
| MAPK10 | 5602 | mitogen-activated protein kinase 10 |
| CD9 | 928 | CD9 molecule |
| ESR1 | 2099 | estrogen receptor 1 |
| FRAP1 | 2475 | mechanistic target of rapamycin (serine/threonine kinase) (MTOR) |
| PML | 5371 | promyelocytic leukemia |
| ABL1 | 25 | c-abl oncogene 1, non-receptor tyrosine kinase |
| TP53 | 7157 | tumour protein p53 |
| LIG4 | 3981 | ligase IV, DNA, ATP-dependent |
| WEE1 | 7465 | WEE1 homolog (S. pombe |
| SYK | 6850 | spleen tyrosine kinase |
| MALT1 | 10892 | mucosa associated lymphoid tissue lymphoma translocation gene 1 |
| PTCH1 | 5727 | patched 1 |
| CASP3 | 836 | caspase 3, apoptosis-related cysteine peptidase |
| BLM | 641 | Bloom syndrome, RecQ helicase-like |
| FYN | 2534 | FYN oncogene related to SRC, FGR, YES |
| WRN | 7486 | Werner syndrome, RecQ helicase-like |

2. The method according to claim 1, wherein the sample comprises a sample of the prostate tumour of the patient.

3. The method according to any one of claims 1 to 2, wherein
each gene in the gene expression profile has a gene expression value and a modified gene expression profile is obtained by multiplying the gene expression value by its marker-factor,
the standardized "good" and "bad" profiles are determined by computing standardized centroids for both "good" and "bad" classes using prediction analysis for microarrays method,
modified class centroids of the marker set are obtained by multiplying the standardized centroids for each class by the marker-factor, and
the modified gene expression profile of the sample is compared to each modified class centroid to determine the tumour is "good" or "bad", wherein the class whose centroid is closest to the modified gene expression profile, in Pearson correlation distance, is predicted to be the class for the sample.

4. The method according to any one of claims 1 to 3, further comprising obtaining an output of the gene expression profile of the sample before comparing the gene expression profile to the standardized "good" and "bad" profiles of the marker set.

5. The method according to any one of claims 1 to 4, wherein the gene expression profile of the sample is determined by screening the sample against a microarray on which gene probes of the marker set are printed.

6. Set of gene probes for predicting prostate cancer risk in a patient having a prostate tumour, consisting of probes for all three gene marker sets as defined in claim 1.

## Patentansprüche

1. Verfahren zur Beurteilung der Wahrscheinlichkeit, dass ein Patient mit Prostatatumor von einer Prostatakrebsbehandlung profitieren wird, wobei das Verfahren umfasst: Gewinnen einer Messenger-RNA-enthaltenden Probe des Prostatatumors des Patienten oder eines Extraktes eines solchen Tumors; Bestimmung eines Genexpressionsprofils der Probe im Hinblick auf Gene von jedem von drei Genmarkersets; und Vergleichen der Genexpressionsprofile der Probe mit standardisierten Profilen für "gut" und "schlecht" jedes einzelnen entsprechenden Genmarkersets, um zu ermitteln, ob die einzelnen Genexpressionsprofile vorhersagen, dass ein Tumor "gut" oder "schlecht" ist,
wobei,
falls alle drei Markersets den Tumor als "gut" prognostizieren, vorhergesagt wird, dass der Patient ein geringes Risiko besitzt und von einer Prostatakrebsbehandlung wahrscheinlich nicht profitieren würde; falls alle drei Markersets den Tumor als "schlecht" prognostizieren, vorhergesagt wird, dass der Patient ein hohes Risiko besitzt und wahrscheinlich von einer Prostatakrebsbehandlung profitieren würde; und falls ein oder zwei der Markersets den Tumor als "gut" prognostizieren oder ein oder zwei der Markersets den Tumor als "schlecht" prognostizieren, vorhergesagt wird, dass der Patient ein mittelmäßiges Risiko besitzt und von einer Prostatakrebsbehandlung möglicherweise oder möglicherweise nicht profitieren wird,
und wobei es sich bei den Genmarkersets um Set 1, Set 2 und Set 3 handelt, wobei
Set 1 aus den folgenden Apoptose-assoziierten Genen besteht:
| Gen | EntrezGene ID | Vollständige Bezeichnung des Gens |
|---|---|---|
| COL4A3 | 1285 | Typ IV-Kollagen |
| BIRC5 | 332 | Baculovirus IAP-Repeat enthaltend 5 |
| TOP2A | 7153 | Topoisomerase (DNA) II alpha |
| CDC2 | 983 | Cyclin-abhängige Kinase 1 (CDK1) |
| NRAS | 4893 | Neuroblastom RAS-Virus (v-ras) Onkogen Homolog |
| GAS1 | 2619 | growth arrest-specific 1 |
| LÍG4 | 3981 | Ligase IV, DNA, ATP-abhängig |
| OSM | 5008 | Oncostatin M |
| PML | 5371 | Promyelozytäre Leukämie |
| TP53 | 7157 | Tumorprotein p53 |
| NF1 | 4763 | Neurofibromin 1 |
| SIAH1 | 6477 | seven in absentia-Homolog 1 (Drosophila) |
| MALT1 | 10892 | Schleimhaut-assoziiertes Lymphgewebe Lymphom-Translokationsgen 1 |
| KIT | 3815 | v-kit Hardy-Zuckerman 4 feline sarcoma viral oncogene-Homolog |
| RHOA | 387 | ras Homolog Genfamilie, Mitglied A |
| ESR1 | 2099 | Östrogenrezeptor 1 |
| RARB | 5915 | Retinsäurerezeptor, beta |
| VAV1 | 7409 | vav 1 Guaninnukleotid-Austauschfaktor |
| WRN | 7486 | Werner-Syndrom, RecQ Helicase-ähnlich |
| TNFRSF10A | 8797 | Tumornekrosefaktor-Rezeptor Superfamilie, Mitglied 10a |
| RIPK1 | 8737 | Rezeptor (TNFRSF)-interagierende Serin-Threoninkinase 1 |
| ABL1 | 25 | c-abl Onkogen 1, Nicht-Rezeptor Tyrosinkinase |
| TERT | 7015 | Telomerase reverse Transcriptase |
| GLI3 | 2737 | GLI Familie Zinkfinger 3 |
| JUN | 3725 | jun Protoonkogen |
| NFKBIA | 4792 | Kernfaktor von kappa light polypeptide gene enhancer in B-Zellen Inhibitor, alpha |
| LCK | 3932 | Lymphozyten-spezifische Proteintyrosinkinase |
| CASP3 | 836 | Caspase 3, Apoptose-verbundene Cysteinpeptidase |
| E2F2 | 1870 | E2F Transkriptionsfaktor 2 |
| LTA | 4049 | Lymphotoxin alpha (TNF Superfamilie, Mitglied 1) |
Set 2 aus den folgenden Zellzyklus-assoziierten Genen besteht:
| Bezeichnung des Gens | EntrezGene ID | Beschreibung |
|---|---|---|
| BCL2 | 596 | B-Zell CLL/Lymphom 2 |
| RAD51 | 5888 | RAD51 Homolog (RecA Homolog, E. coli) (S. cerevisiae) |
| CDKN2B | 1030 | Cyclin-abhängiger Kinaseinhibitor 2B (p15, hemmt CDK4) |
| GML | 2765 | Glycosylphosphatidylinositol-verankertes Molekül-ähnliches Protein |
| E2F1 | 1869 | E2F Transkriptionsfaktor 1 |
| IKZF1 | 10320 | IKAROS Familie Zinkfinger 1 (Ikaros) |
| BLM | 641 | Bloom-Syndrom, RecQ Helicase-ähnlich |
| ABL1 | 25 | c-abl Onkogen 1, Nicht-Rezeptor Tyrosinkinase |
| LIG4 | 3981 | Ligase IV, DNA, ATP-abhängig |
| CCNA2 | 890 | Cyclin A2 |
| NUMA1 | 4926 | Nukleäres Mitoseapparatprotein 1 |
| CCNC | 892 | Cyclin C |
| RBL2 | 5934 | Retinoblastom-ähnlich 2 (p130) |
| LTA | 4049 | Lymphotoxin alpha (TNF Superfamilie, Mitglied 1) |
| ERCC2 | 2068 | excision repair cross-complementing rodent repair deficiency, complementation group 2 |
| CASP3 | 836 | Caspase 3, Apoptose-verbundene Cysteinpeptidase |
| TP53 | 7157 | Tumorprotein p53 |
| RAD54L | 8438 | RAD54-ähnlich (S. cerevisiae) |
| CCND3 | 896 | Cyclin D3 |
| WEE1 | 7465 | WEE1 Homolog (S. pombe) |
| BIRC5 | 332 | Bakulovirus IAP-Repeat enthaltend 5 |
| HDAC1 | 3065 | Histondeacetylase 1 |
Set 3 aus den folgenden mit der Antwort auf externe Reize verbundenen Genen besteht:
| Bezeichnung des Gens | EntrezGene ID | Beschreibung |
|---|---|---|
| COL4A3 | 1285 | Typ IV-Kollagen |
| TOP2A | 7153 | Topoisomerase (DNA) II alpha |
| CDC2 | 983 | Cyclin-abhängige Kinase 1 (CDK1) |
| LYN | 4067 | v-yes-1 Yamaguchi sarcoma viral related oncogene Homolog |
| PXN | 5829 | Paxillin |
| NTRK3 | 4916 | Neurotrophe Tyrosinkinase, Rezeptor, Typ 3 |
| PDGFRA | 5156 | Blutplättchen-Wachstumsfaktorrezeptor, alpha-Polypeptid |
| NRAS | 4893 | Neuroblastom RAS-Virus (v-ras) Onkogen Homolog |
| CHEK1 | 1111 | CHK1 Checkpoint Homolog (S. pombe) |
| PARP1 | 142 | Poly (ADP-Ribose)-Polymerase 1 |
| KIT | 3815 | v-kit Hardy-Zuckerman 4 feline sarcoma viral oncogene-Homolog |
| TGFBR3 | 7049 | Transformierender Wachstumsfaktor, beta-Rezeptor III |
| CCNA2 | 890 | Cyclin A2 |
| NF1 | 4763 | Neurofibromin 1 |
| MAPK10 | 5602 | Mitogen-aktivierte Proteinkinase 10 |
| CD9 | 928 | CD9-Molekül |
| ESR1 | 2099 | Östrogenrezeptor 1 |
| FRAP1 | 2475 | mechanistic target of rapamycin (Serin-/Threoninkinase) (MTOR) |
| PML | 5371 | Promyelozytäre Leukämie |
| ABL1 | 25 | c-abl Onkogen 1, Nicht-Rezeptor Tyrosinkinase |
| TP53 | 7157 | Tumorprotein p53 |
| LIG4 | 3981 | Ligase IV, DNA, ATP-abhängig |
| WEE1 | 7465 | WEE1 Homolog (S. pombe) |
| SYK | 6850 | Milz-Tyrosinkinase |
| MALT1 | 10892 | Schleimhaut-assoziiertes Lymphgewebe Lymphom Translokationsgen 1 |
| PTCH1 | 5727 | patched 1 |
| CASP3 | 836 | Caspase 3, Apoptose-verbundene Cysteinpeptidase |
| BLM | 641 | Bloom-Syndrom, RecQ Helicase-ähnlich |
| FYN | 2534 | FYN Brankogen verwandt mit SRC, FGR, YES |
| WRN | 7486 | Werner-Syndrom, RecQ Helicase-ähnlich |

2. Verfahren nach Anspruch 1, wobei die Probe eine Probe des Prostatatumors des Patienten umfasst.

3. Verfahren nach einem der Ansprüche 1 bis 2, wobei
jedes Gen in dem Genexpressionsprofil einen Genexpressionswert besitzt und ein modifiziertes Genexpressionsprofil erhalten wird, indem man den Genexpressionswert mit seinem Markerfaktor multipliziert,
die standardisierten Profile für "gut"- und "schlecht" durch Berechnung von standardisierten Schwerpunkten für Klassen von "gut" und "schlecht" unter Anwendung der Vorhersageanalyse für Mikroarraymethoden bestimmt werden,
durch Multiplikation der standardisierten Schwerpunkte für jede Klasse mit dem Markerfaktor modifizierte Klassen-Schwerpunkte des Markersets erhalten werden, und
das modifizierte Genexpressionsprofil der Probe mit jedem modifizierten Klassen-Schwerpunkt verglichen wird, um zu bestimmen, ob der Tumor "gut" oder "schlecht" ist, wobei die Klasse, deren Schwerpunkt dem modifizierten Genexpressionsprofil laut Pearson-Korrelationsabstand am nächsten ist, als die Klasse für die Probe vorhergesagt wird.

4. Verfahren nach einem der Ansprüche 1 bis 3, darüber hinaus umfassend das Erhalten einer Ausgabe des Genexpressionsprofils der Probe vor dem Vergleich des Genexpressionsprofils mit den standardisierten Profilen für "gut" und "schlecht" des Markersets.

5. Verfahren nach einem der Ansprüche 1 bis 4, wobei das Genexpressionsprofil der Probe durch Screening der Probe mit einen Microarray mit aufgedruckten Gensonden des Markersets ermittelt wird.

6. Gensonden-Set zur Vorhersage des Prostatakrebsrisikos bei einem Patienten mit einem Prostatatumor, bestehend aus Sonden für alle drei Genmarkersets laut der Definition in Anspruch 1.

## Revendications

1. Méthode d'évaluation de la probabilité pour qu'un patient présentant une tumeur de la prostate bénéficie d'un traitement contre le cancer de la prostate, qui consiste à : obtenir un échantillon ou un prélèvement de la tumeur de la prostate contenant l'ARN messager du patient, déterminer le profil d'expression génétique de l'échantillon pour les gènes de chacun des trois ensembles de marqueurs génétiques, et comparer les profils d'expression génétique de l'échantillon avec les profils « bénins » et « malins » normalisés de chaque ensemble de marqueurs génétiques afin de déterminer si chaque profil d'expression génétique est annonciateur d'une tumeur « bénigne » ou « maligne »,
où,
si les trois ensembles de marqueurs annoncent que la tumeur est « bénigne », il est anticipé que le patient présentera un faible risque et ne bénéficiera probablement pas d'un traitement contre le cancer de la prostate ; si les trois ensembles de marqueurs annoncent que la tumeur est « maligne », il est anticipé que le patient présentera un risque élevé et bénéficiera probablement d'un traitement contre le cancer de la prostate ; et si un ou deux ensembles de marqueurs anticipent que la tumeur est « bénigne » ou si un ou deux ensembles de marqueurs anticipent que la tumeur est « maligne », il est anticipé que le patient présentera un risque intermédiaire et pourra ou non bénéficier d'un traitement contre le cancer de la prostate,
et où les ensembles de marqueurs génétiques sont dénommés Ensemble 1, Ensemble 2 et Ensemble 3, où
L'Ensemble 1 comporte les gènes suivants liés à une apoptose :
| Gène | Saisir le n° du gène | Nom complet du gène |
|---|---|---|
| COL4A3 | 1285 | Collagène de type IV |
| BIRC5 | 332 | Protéine inhibitrice de l'apoptose contenant 5 domaines répétés du baculovirus |
| TOP2A | 7153 | Topoisomérase (ADN) II alpha |
| CDC2 | 983 | Kinase dépendante des cyclines 1 (CDK1) |
| NRAS | 4893 | Homologue oncogène neuroblastome RAS viral (v-ras) |
| GAS1 | 2619 | Spécifique à l'arrêt de croissance 1 |
| LÍG4 | 3981 | ADN ligase IV, ATP dépendante |
| OSM | 5008 | Oncostatine M |
| PML | 5371 | Leucémie aiguë promyélocytaire |
| TP53 | 7157 | Protéine tumorale p53 |
| NF1 | 4763 | Neurofibromine 1 |
| SIAH1 | 6477 | Homologue seven-in-absentia 1 (Drosophile) |
| MALT1 | 10892 | Gène de translocation 1 des lymphomes du tissu lymphoïde associé aux muqueuses |
| KIT | 3815 | Homologue oncogène du virus du sarcome félin v-kit Hardy-Zuckerman 4 |
| RHOA | 387 | Famille de gènes homologues RAS, membre A |
| ESR1 | 2099 | Récepteur oestrogénique 1 |
| RARB | 5915 | Récepteur bêta de l'acide rétinoïque |
| VAV1 | 7409 | Facteur d'échange de nucléotide guanine vav 1 |
| WRN | 7486 | Syndrome de Werner, de type hélicase RecQ |
| TNFRSF10A | 8797 | Superfamille des récepteurs de facteurs de nécrose tumorale, membre 10a |
| RIPK1 | 8737 | Récepteur (TNFRSF) à activité sérine-thréonine kinase 1 |
| ABL1 | 25 | Oncogène c-abl 1, non-récepteur tyrosine kinase |
| TERT | 7015 | Télomérase à activité transcriptase inverse |
| GLI3 | 2737 | Doigt à zinc 3 famille des GLI |
| JUN | 3725 | jun proto-oncogène |
| NFKBIA | 4792 | Facteur nucléaire d'amplificateur kappa de gène polypeptide léger dans l'inhibiteur des lymphocytes B, alpha |
| LCK | 3932 | Protéine tyrosine kinase spécifique aux lymphocytes |
| CASP3 | 836 | Caspase 3, peptidase cystéine liée à l'apoptose |
| E2F2 | 1870 | Facteur 2 de transcription E2F |
| LTA | 4049 | Lymphotoxine alpha (superfamille des TNF, membre 1) |
L'Ensemble 2 comporte les gènes suivants liés àux cycle cellulaire :
| Nom du gène | Saisir le n° du gène | Description |
|---|---|---|
| BCL2 | 596 | LLC/lymphome lymphocytaire 2 |
| RAD51 | 5888 | Homologue RAD51 (homologue RecA, E. coli) (S. cerevisiae) |
| CDKN2B | 1030 | Inhibiteur de la kinase dépendante des cyclines 2B (p15, inhibe CDK4) |
| GML | 2765 | Protéine semblable à une molécule à ancrage glycosylphosphatidylinositol |
| E2F1 | 1869 | Facteur 1 de transcription E2F |
| IKZF1 | 10320 | Doigt à zinc 1 famille des IKAROS (Ikaros) |
| BLM | 641 | Syndrome de Bloom, de type hélicase RecQ |
| ABL1 | 25 | Oncogène c-abl 1, non-récepteur tyrosine kinase |
| LIG4 | 3981 | ADN ligase IV, ATP dépendante |
| CCNA2 | 890 | Cyclines dans A2 |
| NUMA1 | 4926 | Protéine de l'appareil mitotique nucléaire 1 |
| CCNC | 892 | Cyclines dans C |
| RBL2 | 5934 | Type rétinoblastome 2 (p130) |
| LTA | 4049 | Lymphotoxine alpha (superfamille des TNF, membre 1) |
| ERCC2 | 2068 | Réparation par excision qui complète le défaut de réparation des lésions basocellulaires, groupe de complémentation 2 |
| CASP3 | 836 | Caspase 3, peptidase cystéine liée à l'apoptose |
| TP53 | 7157 | Protéine tumorale p53 |
| RAD54L | 8438 | Type RAD54 (S. cerevisiae) |
| CCND3 | 896 | Cycline D3 |
| WEE1 | 7465 | Homologue WEE1 (S. pombe) |
| BIRC5 | 332 | Protéine inhibitrice de l'apoptose contenant 5 domaines répétés du baculovirus |
| HDAC1 | 3065 | Histone-désacétylase 1 |
L'Ensemble 3 comporte les gènes suivants liés à un stimulus externe :
| Nom du gène | Saisir le n° du gène | Description |
|---|---|---|
| COL4A3 | 1285 | Collagène de type IV |
| TOP2A | 7153 | Topoisomérase (ADN) II alpha |
| CDC2 | 983 | Kinase dépendante des cyclines 1 (CDK1) |
| LYN | 4067 | Homologue oncogène lié au virus du sarcome v-yes-1 Yamaguchi |
| PXN | 5829 | Paxilline |
| NTRK3 | 4916 | Tyrosine kinase neurotrophique, récepteur, type 3 |
| PDGFRA | 5156 | Récepteur de facteurs de croissance dérivés des plaquettes, alpha polypeptide |
| NRAS | 4893 | Homologue oncogène neuroblastome RAS viral (v-ras) |
| CHEK1 | 1111 | Homologue checkpoint CHK1 (S. pombe) |
| PARP1 | 142 | poly (ADP-ribose) polymérase 1 |
| KIT | 3815 | Homologue oncogène du virus du sarcome félin v-kit Hardy-Zuckerman 4 |
| TGFBR3 | 7049 | Facteur de croissance de transformation, récepteur bêta III |
| CCNA2 | 890 | Cycline A2 |
| NF1 | 4763 | Neurofibromine 1 |
| MAPK10 | 5602 | Protéine kinase activée par des agents mitogènes 10 |
| CD9 | 928 | Molécule CD9 |
| ESR1 | 2099 | Récepteur oestrogénique 1 |
| FRAP1 | 2475 | Cible mécanistique de la rapamycine (sérine/thréonine kinase) (MTOR) |
| PML | 5371 | Leucémie aiguë promyélocytaire |
| ABL1 | 25 | Oncogène c-abl 1, non-récepteur tyrosine kinase |
| TP53 | 7157 | Protéine tumorale p53 |
| LIG4 | 3981 | ADN ligase IV, ATP dépendante |
| WEE1 | 7465 | Homologue WEE1 (S. pombe) |
| SYK | 6850 | Tyrosine kinase splénique |
| MALT1 | 10892 | Gène de translocation 1 des lymphomes du tissu lymphoïde associé aux muqueuses |
| PTCH1 | 5727 | Patched 1 |
| CASP3 | 836 | Caspase 3, peptidase cystéine liée à l'apoptose |
| BLM | 641 | Syndrome de Bloom, de type hélicase RecQ |
| FYN | 2534 | Oncogène FYN lié à SRC, FGR, YES |
| WRN | 7486 | Syndrome de Werner, de type hélicase RecQ |

2. Méthode selon la revendication 1, où le prélèvement comporte un échantillon de la tumeur de la prostate du patient.

3. Méthode selon la revendication 1 ou 2, où
chaque gène du profil d'expression génétique est associé à une valeur d'expression génétique et où
un profil d'expression génétique modifié est obtenu en multipliant la valeur d'expression génétique par son facteur de marqueurs,
Les profils « bénins » et « malins » normalisés sont déterminés en calculant des centroïdes normalisés pour les catégories « bénin » et « malin » à l'aide d'une analyse prédictive appliquée à la méthode des puces à ADN,
les centroïdes de catégorie modifiés de l'ensemble de marqueurs sont obtenus en multipliant les centroïdes normalisés de chaque catégorie par le facteur de marqueurs, et
le profil d'expression génétique modifié de l'échantillon est comparé à chaque centroïde de catégorie modifié pour déterminer si la tumeur est « bénigne » ou « maligne », où il est anticipé que la catégorie dont le centroïde est le plus proche du profil d'expression génétique modifié, selon le coefficient de corrélation de Pearson, sera la catégorie de l'échantillon.

4. Méthode selon les revendications 1 à 3, qui consiste à obtenir un produit du profil d'expression génétique de l'échantillon avant de comparer le profil d'expression génétique avec les profils « bénins » et « malins » normalisés de l'ensemble de marqueurs.

5. Méthode selon les revendications 1 à 4, où le profil d'expression génétique est déterminé en analysant l'échantillon par rapport à une puce à ADN sur laquelle sont imprimées les sondes génétiques de l'ensemble de marqueurs.

6. Ensemble de sondes génétiques servant à prévoir le risque de cancer de la prostate chez un patient présentant une tumeur de la prostate, qui se compose de sondes associées aux trois ensembles de marqueurs définis dans la revendication 1.
